# EUROPÄISCHE PATENTSCHRIFT

(11) **EP 3 229 862 B1**
(45) Veröffentlichungstag und Bekanntmachung des Hinweises auf die Patenterteilung: **29.01.2020**
(21) Anmeldenummer: 15790519.1
(22) Anmeldetag: 02.11.2015
(51) Int. Cl.: A61M 5/145, A61M 5/315, A61M 5/24

(54) **INJEKTIONSEINRICHTUNG**
INJECTION DEVICE
DISPOSITIF D'INJECTION

(30) Priorität: 12.12.2014 DE 102014225687
(43) Veröffentlichungstag der Anmeldung: 18.10.2017
(73) Patentinhaber: Vetter Pharma-Fertigung GmbH & Co. KG, 88212 Ravensburg (DE)
(72) Erfinder: KÜHNLE, Sarah, 88045 Friedrichshafen (DE); KISTLER, Tobias, 88276 Berg (DE); WURMBAUER, Werner, A-9063 Maria Saal (AT); LAUCHARD, Gerhard, A-9321 Kappel am Krappfeld (AT)
(74) Vertreter: Kordel, Mattias
(86) Internationale Anmeldenummer: PCT/EP2015/075456
(87) Internationale Veröffentlichungsnummer: WO 2016/091474

(56) Entgegenhaltungen:
- WO-A1-95/20145
- WO-A1-2007/094833
- WO-A1-2013/144152
- WO-A2-03/061737

## Beschreibung

Die Erfindung betrifft eine Injektionseinrichtung gemäß Oberbegriff des Anspruchs 1.

Injektionseinrichtungen der hier angesprochenen Art sind bekannt (WO 03/061737 A2; WO 2013/144152 A1; WO 95/20145 A1; WO 2007/094833 A1). Sie weisen einen Kolben zur Verlagerung eines Stopfens in einer Spritze oder Karpule auf, außerdem eine Halterung für den Kolben sowie einen Antrieb zur axialen Verlagerung der Halterung des Kolbens. Mit axial wird hier gemeint, dass der Kolben entlang seiner Längserstreckung bewegt wird und dabei in eine Spritze oder Karpule einfährt, um dort mindestens einen Stopfen zu verlagern. Die Einrichtung weist außerdem eine Steuereinrichtung auf, die mit dem Antrieb zusammenwirkt und dazu dient, die Vorschubgeschwindigkeit des Kolbens und damit des Stopfens innerhalb einer Spritze oder Karpule zu beeinflussen. Vorzugsweise wird der Kolben nach dem Einsetzen einer Spritze oder Karpule in die Injektionseinrichtung bis zum Erreichen einer gewünschten Position mit einer ersten Geschwindigkeit fortbewegt und nach Erreichen dieser Position, nämlich nach Erreichen des Stopfens, angehalten oder mit einer zweiten Geschwindigkeit weiterbewegt, die kleiner ist als die erste Geschwindigkeit. Es hat sich herausgestellt, dass die Injektionseinrichtung in vielen Fällen den Stopfen bereits bewegt, bevor die Vorschubgeschwindigkeit vermindert oder auf Null reduziert werden kann. Dies führt dazu, dass ungewollt eine im Inneren der Spritze oder Karpule vorhandene Substanz ausgetragen werden kann. Dies kann zu einer Schädigung des Benutzers, oder aber zu einem Verlust wertvoller Substanzen führen.

Aufgabe der Erfindung ist es daher, eine Injektionseinrichtung der oben angesprochenen Art zu schaffen, welche diesen Nachteil vermeidet.

Zur Lösung dieser Aufgabe wird eine Injektionseinrichtung vorgeschlagen, welche die in Anspruch 1 genannten Merkmale aufweist. Sie umfasst eine Sensoreinrichtung, welche die Relativposition des Kolbens oder zumindest eines Teilelements desselben gegenüber der Halterung erfasst. Die Sensoreinrichtung weist wenigstens ein mit der Sensoreinrichtung zusammenwirkendes Sensorelement auf. Die Halterung ist gegenüber dem Kolben oder zumindest einem Teilelement desselben relativbeweglich ausgebildet. Dabei erfolgt die Relativbewegung entgegen einer Vorspannkraft und zwar über eine begrenzte Strecke. Während sich die Halterung in Richtung auf den Stopfen in der Spritze oder Karpule zu bewegt, kann der Kolben oder ein Teilelement durch einen Stopfen in einer Spritze oder Karpule angehalten angehalten werden, während die Halterung sich weiterbewegt. Der Anschlag des Kolbens oder eines Teilelements am Stopfen kann mittels des Sensorelements bereits erfasst werden, bevor nachhaltige Kräfte des Kolbens oder des Teilelements auf den mindestens einen Stopfen innerhalb der Spritze oder Karpule ausgeübt werden. Dadurch kann sichergestellt werden, dass dieser, auch wenn er nur mit geringen Haltekräften innerhalb der Spritze oder Karpule positioniert ist, durch die Injektionseinrichtung nicht ungewollt verlagert wird. Die Injektionseinrichtung zeichnet sich dadurch aus, dass die Steuereinrichtung so ausgelegt ist, dass bei Aktivierung des Sensorelementes der Antrieb der Injektionseinrichtung so beeinflusst wird, dass die Vorschubgeschwindigkeit des Kolbens zumindest reduziert wird. Dadurch wird sichergestellt, dass ein innerhalb einer Spritze oder Karpule angeordneter Stopfen nach Beendigung einer Relativbewegung des Kolbens oder eines Teilelements gegenüber der Halterung nicht ungewollt mit hoher Vorschubgeschwindigkeit aus seiner Ausgangsposition verlagert wird.

Durch die hier gewählte Ausgestaltung der Injektionseinrichtung wird also erreicht, dass bei bei deren Verwendung zunächst die Halterung gemeinsam mit dem Kolben oder einem Teilelement derselben in Richtung auf den mindestens einen Stopfen einer Spritze oder Karpule verlagert wird. Bei Berührung des mindestens einen Stopfens bleibt der Kolben oder das Teilelement stehen, während die Halterung sich weiterbewegt. Es ergibt sich dadurch eine Relativbewegung zwischen Halterung und Kolben oder dessen Teilelement, die von dem Sensorelement erfasst wird. Während dieser Relativbewegung kann die Vorschubgeschwindigkeit der Halterung zumindest reduziert werden auf ein gewünschtes Maß oder auch auf Null.

Bei einem bevorzugten Ausführungsbeispiel der Injektionseinrichtung ist vorgesehen, dass das Sensorelement einen Mikroschalter umfasst. Diese brauchen nur einen sehr kleinen Bauraum, sodass die Injektionseinrichtung kompakt realisiert werden kann. Überdies sind derartige Schalter sehr funktionssicher.

Erfindungsgemäß ist vorgesehen, dass der Kolben einen Grundkörper umfasst und dass das Teilelement gegenüber diesem verlagerbar ist. Das Teilelement kann sehr leicht ausgebildet sein, sodass aufgrund seiner geringen Massenträgheit eine sehr schnelle Verlagerung erfolgen kann, die Ansprechzeit der Injektionseinrichtung sehr kurz ist und die auf den Stopfen wirkenden Kräfte während der Relativbewegung sehr klein sind. Weiter ist erfindungsgemäß vorgesehen, dass der Grundkörper des Kolbens als ein einen Innenraum umfassender Hohlzylinder ausgebildet ist, und dass in dem Innenraum das als Schaltstößel ausgebildete Teilelement verschieblich angeordnet ist.

Weitere Ausgestaltungen und Vorteile der Injektionseinrichtung ergeben sich aus den übrigen Unteransprüchen.

Die Erfindung wird im Folgenden anhand der Zeichnungen näher erläutert. Es zeigen:
- Figur 1: eine perspektivische Ansicht eines Teils eines ersten Ausführungsbeispiels einer Injektionseinrichtung;
- Figur 2: einen Längsschnitt durch Teile der in Figur 1 wiedergegebenen Injektionseinrichtung;
- Figur 3: eine vergrößerte Darstellung einer Halterung mit einem Schalter der Injektionseinrichtung nach den Figuren 1 und 2 in einer ersten Schaltstellung;
- Figur 4: eine vergrößerte Darstellung des in Figur 3 wiedergegebenen Schalters in einer zweiten Schaltstellung;
- Figur 5: ein nicht zur Erfindung gehörendes Beispiel von Teilen einer Injektionseinrichtung im Längsschnitt;
- Figur 6: eine vergrößerte Darstellung einer Haltung mit einem Schalter der Injektionseinrichtung nach Figur 5 in einer ersten Schaltstellung und
- Figur 7: eine vergrößerte Darstellung des Schalters gemäß Figur 6 in einer zweiten Schaltstellung.

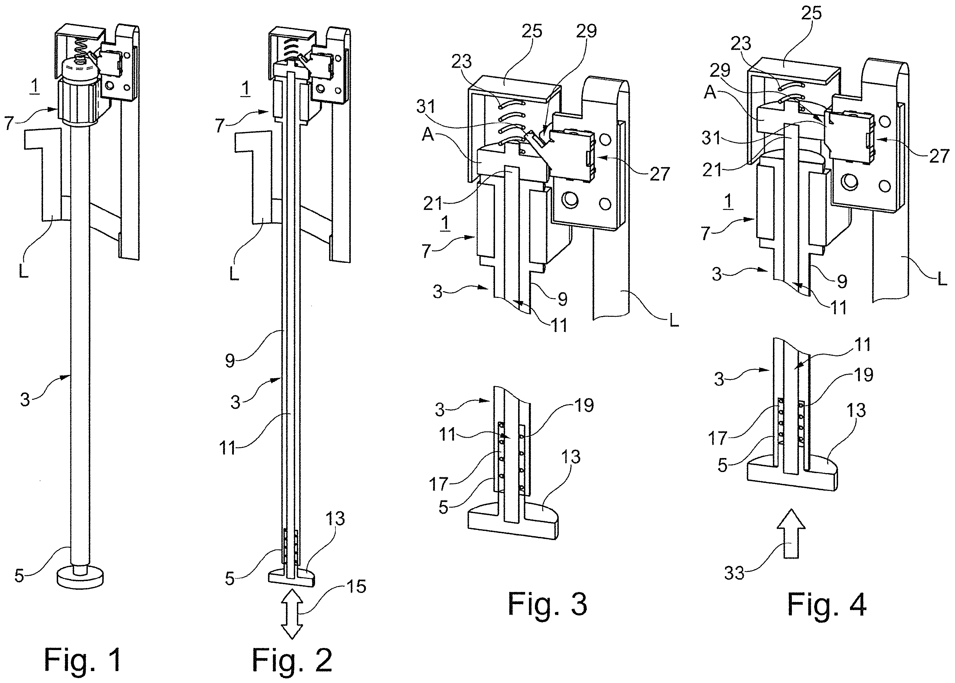

Figur 1 gibt Teile eines ersten Ausführungsbeispiels einer nicht als Ganzes dargestellten Injektionseinrichtung 1 wieder, nämlich einen Kolben 3, der dazu dient, mit seinem in Figur 1 unteren Ende einen in einer hier nicht dargestellten Spritze oder Karpule vorhandenen Stopfen zu verlagern, sei es um eine Medikament auszutragen und einem Patienten zu verabreichen oder um in einer Zweikammerkarpule vorhandene Substanzen miteinander zu mischen, um ein verabreichungsfertiges Medikament zu erhalten. Der Kolben 3 ist an einer Halterung 7 angebracht, die innerhalb der Injektionseinrichtung 1 in Richtung der Längserstreckung des Kolbens 3 entlang einer hier nicht dargestellten Führungsschiene oder dergleichen verlagerbar ist. Beispielhaft wird hier in Figur 1 davon ausgegangen, dass sich die Halterung 7 in einer oberen Aufgangsposition befindet und nach unten verlagert wird, um einen Stopfen zu verlagern.

Figur 2 zeigt die in Figur 1 wiedergegebenen Elemente einer Injektionseinrichtung 1 im Längsschnitt. Gleiche Teile sind mit gleichen Bezugsziffern versehen, sodass insofern auf die Beschreibung von Figur 1 verwiesen wird. In der Schnittdarstellung wird erkennbar, dass der Kolben 3 dieses Ausführungsbeispiels einen Grundkörper 9 und ein gegenüber diesem verlagerbares Teilelement 11 aufweist, dabei ist der Grundkörper 9 als ein einen Innenraum aufweisender Hohlzylinder ausgebildet, in dem das hier als Schaltstößel ausgebildete Teileelement 11 verschieblich angeordnet ist. Der Grundkörper 9 ist lagefest, das heißt also bezüglich der Längserstreckung des Kolbens 3 unverschieblich mit der Halterung 7 verbunden. Wird also die Halterung 7 auf und ab bewegt, bewegt sich mit dieser der Kolben 3, somit dessen Grundkörper 9 synchron mit der Halterung 7 auf und ab.

Aus Figur 2 ist ersichtlich, dass der Schaltstößel, also das Teilelement 11, über das untere Ende 5 des Grundkörpers 9 hinausragt und an seinem unteren Ende hier eine Platte 13 aufweist, die auf einem zu verlagernden Stopfen aufliegt, wenn die Halterung 7 gemeinsam mit dem Kolben 3 gemäß dem Doppelpfeil 15 nach unten in das Innere einer Spritze oder Karpule hineinbewegt wird.

Eine Aufwärtsbewegung der Halterung 7 wird insbesondere dann veranlasst, wenn das in der Spritze oder Karpule vorhandene Medikament vollständig verabreicht wurde und eine neue Spritze oder Karpule in die Injektionseinrichtung eingesetzt werden soll. In der Regel wird der Stopfen nach jeder Injektion entlastet, indem die Halterung 7 mit der Kolbenstange 3 etwas zurück, also nach oben, verlagert wird.

Figur 3 zeigt in vergrößerter Darstellung einerseits den Bereich der Halterung 7 der Injektionseinrichtung 1 und andererseits das untere Ende 5 des Kolbens 3 mit der an dem als Schaltstößel ausgebildeten Teilelement 11 angebrachten Platte 13. Gleiche Teile sind mit gleichen Bezugsziffern versehen, sodass insofern auf die vorangegangenen Figuren und Beschreibungen verwiesen wird.

Aus der vergrößerten Wiedergabe des unteren Endes 5 des Kolbens 3 ist ersichtlich, dass dessen Grundkörper 9 zum einen einen Innenraum zur Aufnahme des zweiten Teilelements 11, also des Schaltstößels, aufweist und zum anderen einen vergrößerten Bereich 17, welcher der Aufnahme eines als Schraubenfeder ausgebildeten Vorspannelementes 19 dient. Dieses stützt sich an einem oberen Rand des Bereiches 17 und zum anderen an der Platte 13 unter einer Vorspannung ab, sodass diese in einem maximalen Abstand zum unteren Ende 5 des Kolbens 3 gehalten wird, wobei das Teilelement 11 also mit einer in Figur 3 nach unten gerichteten Kraft beaufschlagt wird. Auf geeignete Weise wird verhindert, dass das zweite Teilelement 11 durch das Vorspannelement 19 nach unten aus dem Grundkörper 9 herausgeschoben wird. Bei dem hier dargestellten Ausführungsbeispiel ist an einem oberen Ende 21 des Teilelements 11 ein Anschlag A vorgesehen, der hier beispielhaft ringförmig ausgebildet und so groß ist, dass er nicht durch den Innenraum des Grundkörpers 9 hindurch nach unten verlagert werden kann. Der Anschlag A stützt sich an der Oberseite der Halterung 7 ab, sodass das Vorspannelement 19 das zweite Teilelement 11 mit der Platte 13 nur über eine vorbestimmten Weg nach unten herausdrücken kann.

Auf das obere Ende 21 des Teilelements 11 wirkt ein Federelement 23, welches hier beispielhaft wiederum als Schraubenfeder ausgelegt ist und sich unter Vorspannung einerseits an dem oberen Ende 21 und andererseits an einem Widerlager 25 der Halterung 7 abstützt. Durch die Vorspannung des Federelementes 23 wird das zweite Teilelement 11 - wie auch schon durch das Vorspannelement 19 - nach unten gegenüber dem fest mit der Halterung 7 verbundenen Grundkörper 9 der Kolbenstange 3 nach unten gedrückt.

Bei entsprechender Auslegung kann auf das Vorspannelement 19 verzichtet werden. Entsprechend ist es möglich, nur das Vorspannelement 19 und nicht das Federelement 23 vorzusehen.

Die Injektionseinrichtung 1 weist außerdem eine Sensoreinrichtung 27 auf, die ein Sensorelement 29 umfasst, welches hier einen Mikroschalter und einen im Bewegungspfad des oberen Endes 21 des zweiten Teilelements 11 liegenden Schaltarm 31 aufweist. Dieser ist so angeordnet, dass er aus seiner in Figur 3 dargestellten Ausgangsposition beziehungsweise ersten Schaltstellung bei einer nach oben gerichteten Verlagerung des zweiten Teilelements 11, also des Schaltstößels, in eine zweite Position beziehungsweise zweite Schaltstellung verschwenkt wird.

Grundsätzlich ist es möglich, die Sensoreinrichtung mit dem Sensorelement 29 beziehungsweise dem Mikroschalter am oberen Ende 21 des zweiten Teilelements 11 vorzusehen und dafür Sorge zu tragen, dass der Schaltarm 31 mit einem fest an der Halterung 7 angebrachten Widerlager zusammenwirkt. Der hier vorgesehene Aufbau zeichnet sich allerdings dadurch aus, dass das Sensorelement 29 lagefest an der Halterung 7 angebracht ist und dass dessen Schaltarm 31 von dem Teilelement 11, welches relativbeweglich gegenüber dem Grundkörper 9 des Kolbens 3 ausgebildet ist, betätigt wird. Der Kolben 3 ist dadurch leichter.

Aus den Erläuterungen wird deutlich, dass, statt des Mikroschalters oder zusätzlich, andere Einrichtungen, wie Magnetbänder, Lichtschranken oder dergleichen, eingesetzt werden können, um eine bestimmte Position des Teilelements 11 gegenüber der Halterung 7 zu erfassen.

Figur 4 zeigt wiederum einen vergrößerten Ausschnitt der Injektionseinrichtung 1, nämlich das obere Ende 21 des Kolbens 3 mit der Halterung 7 und das untere Ende 5 des Kolbens 3 mit der Platte 13. Gleiche Teile sind mit gleichen Bezugsziffern versehen, sodass auf die Beschreibung der vorangegangenen Figuren verwiesen wird.

Die Halterung 7 wird gemeinsam mit dem Kolben 3 nach unten verlagert in eine Spritze oder Karpule hinein, bis die Platte 13 auf einen Stopfen in einer Spitze oder Karpule stößt, sodass sie entsprechend dem Pfeil 33 die Platte 13 entgegen der Vorspannung des Vorspannelementes 19 gegenüber dem Grundkörper 9 des Kolbens 3 nach oben verlagert wird. Bei dieser Verlagerung wirkt auch das Federelement 23 auf die Platte 13. Unmittelbar nachdem die Platte 13 einen Stopfen einer Spritze oder Karpule berührt, findet die beschriebene Relativbewegung des mit der Platte 13 verbundenen Teilelements 11 gegenüber dem Grundkörper 9 des Kolbens 3 statt, während sich die Halterung 7 noch weiter in Richtung auf den Stopfen bewegt. Die Aufwärtsbewegung der Platte 13 und des ersten Teilelements 11 unter Einwirkung einer durch den Pfeil 33 angedeuteten Kraft wird dadurch beendet, dass die Platte 13 an einem Anschlag, hier am unteren Ende 5 des Kolbens 3 anschlägt, wie dies Figur 4 zeigt.

Aus den Figuren 1 bis 4 ist noch ersichtlich, dass die elektrische Versorgung der Sensoreinrichtung 27 sowie des Sensorelementes 29 über eine Leiterbahn L erfolgt, die flexibel ausgebildet ist, sodass sie der Halterung 7 folgen kann, während diese gemeinsam mit dem Kolben 3 innerhalb der Injektionseinrichtung 1 in Richtung der Längserstreckung des Kolbens 3 verfahren wird.

Die Leiterbahn L ist einerseits mit einer Energieversorgung verbunden, sie dient andererseits dazu, Signale des Sensorelementes 29, hier also Schaltsignale des Mikroschalters an eine hier nicht dargestellte Steuereinrichtung weiterzuleiten, mit deren Hilfe der die Aufwärts- und Abwärtsbewegung der Halterung 7 bewirkende Antrieb beeinflusst wird.

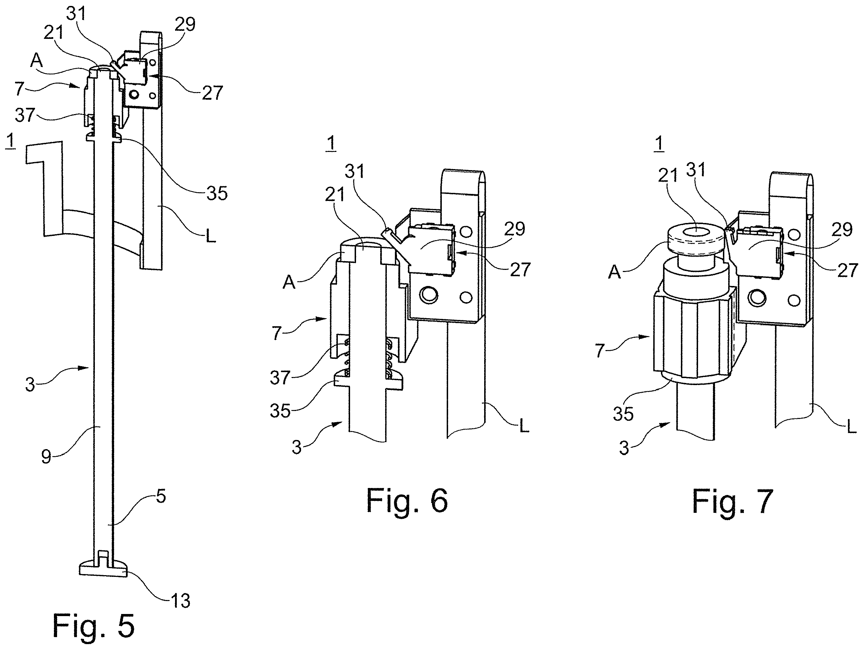

Figur 5 zeigt ein nicht zur Erfindung gehörendes Beispiel der in Figur 1 dargestellten Elemente einer Injektionseinrichtung 1. Gleiche und funktionsgleiche Teile sind mit gleichen Bezugsziffern versehen.

Figur 5 entspricht der Darstellung gemäß Figur 2: Der Kolben 3 der Injektionseinrichtung 1 ist, ebenso wie die Halterung 7 im Längsschnitt dargestellt. Der Kolben 3 ist hier als Vollelement ausgebildet, also nicht hohl. Er trägt an seinem unteren Ende 5 eine Platte 13, die hier, wie bei dem oben dargestellten Ausführungsbeispiel, nicht zwingend erforderlich ist, aber dazu dient, dass das untere Ende 5 den in einer Spritze oder Karpule vorhandenen Stopfen gut erfasst, beispielsweise auch dann, wenn er mit einer zentralen Bohrung versehen sein sollte.

Im Unterschied zum Ausführungsbeispiel gemäß Figur 1 ist der Kolben 3 selbst innerhalb der Halterung 7 verschieblich gelagert. Er kann also in Richtung seiner Längserstreckung eine Relativbewegung innerhalb der Halterung 7 und gegenüber dieser durchführen. Der Kolben 3 ist im Bereich seines oberen Endes 21 und unterhalb der Halterung 7 mit einem Widerlager, hier einer umlaufenden Schulter 35 versehen, an der sich ein Federelement 37 abstützt, welches hier als Schraubenfeder ausgebildet ist, um den Grundkörper 9 des Kolbens 3 verläuft, sich bis in das Innere der Halterung 7 erstreckt und an dieser abstützt. Das Federelement 37 steht unter Vorspannung, sodass es eine nach unten gerichtete Kraft auf den Kolben 3 ausübt. Auf geeignete Weise wird verhindert, dass der Kolben 3 nach unten aus der Halterung 7 herausrutscht. Hier ist beispielswahrhaft ein vorzugsweise ringförmiger Anschlag A vorgesehen, der am oberen Ende 21 oberhalb der Halterung 7 angebracht ist, sodass das obere Ende 21 nicht durch das Innere der Halterung 7 nach unten ausweichen kann, auch wenn das Federelement 37 eine Vorspannung auf den Kolben 3 ausübt.

Die umlaufende Schulter 35 an dem Kolben 3 ist so ausgebildet, dass zwar eine Relativbewegung zwischen der Halterung 7 und dem Kolben 3 möglich ist, dass aber der Weg dieser Bewegung begrenzt ist. Die Halterung 7 kann sich gegenüber dem Kolben 3 nur soweit nach unten verlagern, bis sie an einem Anschlag, hier an der Schulter 35 anschlägt.

Auch bei dem in Figur 5 dargestellten Beispiel der Injektionseinrichtung 1 ist eine Sensoreinrichtung 27 vorgesehen, die ein Sensorelement 29 umfasst, welches hier wiederum vorzugsweise einen Mikroschalter mit einem Schaltarm 31 umfasst. Der Schaltarm 31 ist, wie bei dem oben beschriebenen Ausführungsbeispiel, in dem Bewegungsweg des Kolbens 3 angeordnet ist und zwar derart, dass er bei einer Relativbewegung des Kolbens 3 gegenüber der Halterung 7 durch das obere Ende 21 des Kolbens 3 nach oben der Schaltarm 31 ausgelenkt wird und das Sensorelement 29 damit anspricht.

Figur 6 zeigt die Halterung 7 und das obere Ende 21 des Kolbens 3 der Injektionseinrichtung 1 in vergrößerter Darstellung in ersten Funktionsstellung. Gleiche Teile sind mit gleichen Bezugsziffern versehen, sodass auf die vorangegangene Beschreibung verwiesen wird.

Aus Figur 6 ist nochmal deutlich erkennbar, dass das Federelement 37 sich einerseits im Inneren der Halterung 7 abstützt und andererseits in dem Widerlager des Kolbens 3, welches hier als umlaufende Schulter 35 ausgebildet ist. Das Federelement 37 drückt aufgrund seiner Vorspannung den Kolben 3 in seine, durch den Anschlag A am oberen Ende 21 des Kolbens 3 definierte unterste Position. Diese nimmt der Kolben 3 dann ein, wenn von unten keine Kraft auf ihn wirkt. In dieser Funktionsstellung ist der Schaltarm 31 nicht betätigt.

Figur 7 zeigt die in Figur 6 wiedergegebenen Elemente der Injektionseinrichtung 1 in einer zweiten Funktionsstellung. Gleiche Teile sind mit gleichen Bezugsziffern versehen, sodass auf die vorangegangene Beschreibung verwiesen wird. Anders als in Figur 6 ist die Halterung 7 allerdings nicht im Längsschnitt, sondern in Seitenansicht wiedergegeben, ebenso der Kolben 3.

Es zeigt sich, dass bei einer Abwärtsbewegung der Halterung 7, welcher der Kolben 3 folgt, dieser auf einen Stopfen in einer Spritze oder Karpule auflaufen kann, sodass eine von unten wirkende Kraft auf den Kolben 3 wirkt, was durch den Pfeil 33 angedeutet wird.

Durch diese Kraft wird der Kolben 3 gegenüber der Halterung 7 nach oben verlagert und zwar gegen die Vorspannkraft des Federelementes 37, welches bei der Relativbewegung des Kolbens 3 gegenüber der Halterung 7 komprimiert wird. Dabei wird auch das obere Ende 21 des Kolbens 3 gemeinsam mit dem Anschlag A nach oben bewegt, sodass der Schaltarm 31 des Sensorelementes 29, welches hier als Mikroschalter ausgebildet ist, der Sensoreinrichtung 27 nach oben verschwenkt wird. Er gelangt also aus der in Figur 6 dargestellten ersten Schaltposition in die in Figur 7 wiedergegebene zweite Schaltposition. Das Sensorelement 29 spricht also an, sodass ein entsprechendes Signal über die Leiterbahn L an die hier nicht dargestellte Steuereinrichtung des Antriebs abgegeben wird. Es wird deutlich, dass auch statt des Mikroschalters oder zusätzlich zu diesem andere Einrichtungen verwendet werden können, wie dies auch in Zusammenhang mit den Erläuterungen zu Figur 3 festgehalten wurde.

Die Aufwärtsbewegung des Kolbens 3 gegenüber der Halterung 7 wird, wie gesagt, begrenzt, nämlich dadurch, dass die Schulter 35 von unten an der Halterung 7 anschlägt.

Im Folgenden wird auf die Funktion der Injektionseinrichtung 1, die von den Elementen in den Figuren 1 bis 7 dargestellt sind, näher eingegangen:
Injektionseinrichtungen 1 der hier beschriebenen Art dienen dazu, mittels eines Kolbens 3 einen in einer Spritze oder Karpule vorhandenen Stopfen zu bewegen und zwar in der Regel derart, dass sich das Innenvolumen der Spritze beziehungsweise Karpule verringert und darin vorhandene Substanzen ausgetragen werden. Dazu werden eine Spitze oder einer Karpule in die Injektionseinrichtung 1 eingesetzt und derart ausgerichtet, dass sie koaxial zum Kolben 3 positioniert sind, sodass der Kolben 3 in das Innere der Spritze oder Karpule einfahren und den Stopfen verlagern kann.

Injektionseinrichtungen 1 der hier angesprochenen Art werden auch in Zusammenhang mit bekannten Doppelkammerkarpulen verwendet, bei denen üblicherweise der Innenraum von einem Endstopfen dichtend verschlossen wird und ein zweiter sogenannter Mittelstopfen den Innenraum in zwei Teilräume unterteilt, in denen verschiedene medizinische Substanzen unter anderem auch für Injektionen bestimmtes Wasser vorhanden sein kann. Bei Doppelkammerkarpulen wird zunächst der endständige Stopfen um einen bestimmten Weg verschoben, wobei ein Überdruck in dem Teilraum zwischen diesem Stopfen und dem Mittelstopfen entsteht, sodass der Mittelstopfen in dieselbe Richtung bewegt wird, wie der Endstopfen. Schließlich gelangt der Mittelstopfen in den Bereich eines Bypasses auf der Innenfläche der Karpule, sodass sich die beiden in den getrennten Teilräumen vorhandenen Substanzen mischen können.

Üblicherweise ist vorgesehen, dass sich der Kolben 3 der Injektionseinrichtung 1 in seiner maximal zurückgezogenen Position befindet. Dadurch kann die Spritze oder Karpule leicht in die Injektionseinrichtung 1 eingesetzt werden. Dann wird der Kolben 3 gemeinsam mit der Halterung 7 so verlagert, dass das untere Ende 5 des Kolbens 3 gegebenenfalls eine hier vorhandene Platte 13 in Richtung auf den wenigstens einen Stopfen in der Spritze oder Karpule bewegt wird. Die Vorschubgeschwindigkeit ist dabei vergleichsweise groß, um in dieser Betriebsphase der Injektionseinrichtung 1 Zeit zu sparen. Üblicherweise ist vorgesehen, dass der Kolben 3, sobald er den Stopfen in der Spritze oder Karpule erstmalig berührt, sofort angehalten oder mit reduzierter Geschwindigkeit weiterbewegt wird. Dazu ist es erforderlich, den Moment zu erfassen, in dem der Kolben 3 den Stopfen berührt. Aufgrund technischer Gegebenheiten bekannter Injektionseinrichtungen ist es häufig so, dass selbst bei einer sofortigen Inaktivierung des Antriebs der Halterung 7 sich diese gemeinsam mit dem Kolben 3 in der ursprünglichen Bewegungsrichtung weiterbewegt und eine Kraft auf den Stopfen ausgeübt wird. Dieser wird dadurch unkontrolliert verlagert. Dadurch kann eine in einer Spritze oder Karpule vorhandene Substanz ausgetragen werden.

Bei den hier dargestellten Injektionseinrichtungen 1 ist vorgesehen, dass die Halterung 7 eine Relativbewegung gegenüber dem Kolben 3 oder dessen Teilelement 11 durchführen kann, sobald der Kolben 3 oder das Teilelement 11 durch den Stopfen angehalten werden. Der Kolben 3 beziehungsweise das Teilelement 11 ist mit einer Vorspannkraft beaufschlagt, die ihn/es gegenüber der Halterung 7 in einer definierten Position hält. Die Vorspannkraft ist so ausgelegt, dass die auf den Stopfen wirkenden Kräfte nicht ausreichen, diesen zu verlagern.

Werden die Halterung 7 und der Kolben 3 aus einer Ausgangslage in einem Abstand zu einer Spritze oder Karpule in Richtung auf deren Stopfen bewegt, so werden beide, also die Halterung 7 und der Kolben 3, der auch ein Teilelement 11 umfassen kann, zunächst synchron in die Spritze beziehungsweise die Karpule eingefahren. Sobald der Kolben 3 oder ein Teilelement 11 desselben mit dem Stopfen der Spritze oder Karpule in Berührung tritt und nicht weiter verlagert wird, wird die Halterung 7 gegenüber dem stillstehenden Kolben 3, wie in den Figuren 5 bis 7 erläutert, gegen eine Vorspannkraft verlagert. Entsprechendes findet bei dem Ausführungsbeispiel gemäß den Figuren 1 bis 4 statt: Dort wird die Halterung 7 gegenüber einem Teilelement 11 des Kolbens 3, welches hier als Schaltstößel ausgebildet ist, bei Anlage an dem Stopfen relativ bewegt.

Bei beiden Injektionseinrichtungen 1 bleibt also ein Teilelement 11 des Kolbens 3 beziehungsweise der Kolben 3 selbst stehen, sobald der Stopfen berührt wird. Eine weitere Vorwärtsbewegung des Teilelements 11 beziehungsweise des Kolbens 3 findet auch dann nicht statt, wenn sich die Halterung 7 weiter in Richtung auf den Stopfen bewegt, weil die auf den Kolben 3 oder das Teilelement 11 ausgeübte Vorspannkraft so gering ausgelegt ist, dass durch diese allein der Stopfen nicht verlagert wird.

Sobald der Kolben 3 oder dessen Teilelement 11 bei einer Vorschubbewegung der Haltung 7 den Stopfen in der Spritze oder Karpule berührt und anhält, wird das Sensorelement 29, hier also ein Schaltarm 31 eines Mikroschalters aus einer ersten Schaltposition in eine zweite Schaltposition verlagert, sodass ein Schaltsignal an die Steuereinrichtung übertragen wird, welche mit dem Antrieb zusammenarbeitet. Durch dieses Signal wird der Antrieb entweder inaktiviert, das heißt, ein Motor, insbesondere Elektromotor, wird durch Abschalten des Stroms, gegebenenfalls durch Kurzschluss, sofort angehalten. Möglich ist es auch, um die Vorschubbewegung der Haltung 7 möglichst rasch zu beenden, eine Drehrichtungsumkehr des Motors zu bewirken. Trotz der Massenträgheit des Halters 7 und der Reaktionsträgheit des Antriebs übt die Halterung 7 noch keine Kraft auf den Stopfen innerhalb der Spritze oder Karpule aus, weil zunächst eine Relativbewegung der Halterung 7 gegenüber einem Teilelement 11 oder dem Kolbens 3 erfolgt und von dieser keine Kraft auf den Stopfen ausgeübt wird. Während der Relativbewegung der Halterung 7 gegenüber dem Teilelement 11 beziehungsweise dem Kolben 3 wirkt lediglich die Vorspannkraft eines Federelementes oder eines Vorspannelementes auf das den Stopfen berührende Teil, also den Kolben 3 beziehungsweise dessen Teilelement 11. Diese Vorspannkraft ist durch Dimensionierung des Federelementes beziehungsweise Vorspannelementes so gering, dass auch sehr leicht innerhalb einer Spritze oder Karpule verschiebliche Stopfen, die nur durch Reibungskräfte in Ihrer Ausgangsposition gehalten werden, durch diese Vorspannkraft nicht innerhalb der Spritze oder Karpule verlagert werden.

Die Relativbewegung der Halterung 7 gegenüber dem Kolben 3 beziehungsweise dessen Teilelements 11 führt dazu, dass das obere Ende 21 des Kolbens 32 beziehungsweise des Teilelements 11 mit dem Sensorelement 29 zusammenwirkt. Hier ist vorgesehen, dass das obere Ende 21, vorzugsweise der dort vorgesehene Anschlag A, mit einem Schaltarm 31 des als Mikroschalter ausgebildeten Sensorelementes 29 zusammenwirkt. Der Schaltarm 31 wird aus seiner ersten Schaltstellung wie sie in den Figuren 3 und 6 dargestellt ist, beim Auflaufen des Kolbens 3 oder des Teilelements 11 betätigt und in seine zweite Schaltstellung verlagert, die sich aus den Figuren 4 und 7 ergibt. Dadurch wird ein Signal an die Steuereinheit und über diese an den Antrieb der Injektionseinrichtung abgegeben. Sobald dies der Fall ist, wird der Antrieb inaktiviert und der Vorschub der Halterung 7 zumindest reduziert oder gestoppt.

Oben wurde ausgeführt, dass eine Relativbewegung zwischen Halter 7 und Kolben 3 beziehungsweise an dem Teilelement desselben über eine begrenzte Strecke möglich ist, nämlich bis der Kolben 3 beziehungsweise dessen Teilelement 11 den zugehörigen Anschlag erreicht hat. Die zum Erreichen des Anschlags erforderliche Zeit reicht, die Vorschubgeschwindigkeit der Halterung 7, wie gewünscht, zumindest zu reduzieren. Erst bei Erreichen des Anschlags übt die Halterung 7 eine Kraft auf den Stopfen in der Spritze oder Karpule aus, die größer ist als die auf den Kolben 3 beziehungsweise dessen Teilelement 11 wirkende Vorspannkraft.

Es ist nun möglich, dass ein Anwender eine weitere Bewegung der Halterung 7 in Richtung der vorherigen Bewegung veranlasst, um bei Verwendung einer Doppelkammerkarpule zunächst das Mischen der in den Teilräumen der Doppelkammerkarpule vorhanden Substanz zu bewirken, also um ein in der Karpule vorhandenes Medikament zu primen. Anschließend kann der Anwender dann durch erneute Aktivierung des Antriebs und des Vorschubs der Halterung 7 die Injektion des fertigen Medikamentes veranlassen.

Bei einem anderen Ausführungsbeispiel der Injektionseinrichtung ist die Steuereinrichtung des Antriebs so ausgelegt, dass nach Erhalt eines Schaltsignales von dem Sensorelement zunächst ein automatischer weiterer Vorschub der Halterung 7 beziehungsweise des Kolbens 3 und dessen Teilelements 11 erfolgt, bevorzugt mit reduzierter Geschwindigkeit, um ein Priming des in einer Doppelkammerkarpule vorhandenen Medikamentes automatisch durchzuführen. Wenn dies erfolgt ist, wird die Halterung 7 erst dann wieder aktiviert, wenn der Anwender dies wünscht und die Injektionseinrichtung 1 entsprechend aktiviert.

In der Regel erfolgt also ein relativ rascher Vorschub der Halterung 7 mit dem Kolben 3 und dem gegebenenfalls vorgesehene Teilelement 11. Sobald ein Stopfen erfasst wird, was durch das Sensorelement 29 geschieht, wird der Vorschub der Halterung 7 zunächst gestoppt. Während des nun erfolgenden Primings eines Medikamentes in einer Doppelkammerkarpule erfolgt ein definierter langsamer Vorschub der Halterung gemeinsam mit dem Kolben und gegebenenfalls dessen Teilelements, bis ein Mittelstopfen in der Doppelkammerkarpule in den Bereich eines Beipasses gelangt. Durch einen weiteren Vorschub wird der Priming-Prozess durchgeführt, bei dem die in den Teilräumen der Doppelkammerkarpule vorhandenen Substanzen gemischt und/oder aktiviert werden. Während des Mischens beziehungsweise Aktivierens der Substanzen wird der Vorschub vorzugsweise gestoppt. Danach wird der Antrieb der Halterung 7 wieder aktiviert, um einen Vorschub des Stopfens innerhalb der Karpule durch den Kolben 3 oder dessen Teilelement 11 in einer gewünschten Geschwindigkeit zu bewirken, um das fertige Medikament zu verabreichen.

Die Möglichkeit der Relativbewegung zwischen Halterung 7 und dem Kolben (Figuren 5 bis 7) beziehungsweise eines Teilelements 11 des Kolbens 3 (siehe Figuren 1 bis 4) hat zur Folge, dass die Injektionseinrichtung 1 während einer ersten Zeitphase nach Berührung des Stopfens innerhalb einer Spritze oder Karpule noch keine wesentlichen Kräfte auf diesen Stopfen ausübt. Die von der Halterung 7 aufgebrachten Kräfte wirken erst dann auf den Stopfen, wenn die Halterung 7 über einen definierten Weg eine Relativbewegung gegenüber dem Kolben 3 oder einem Teilelement 11 desselben durchlaufen hat. Die dafür erforderliche Zeit wird genutzt, den Antrieb der Halterung 7 zu stoppen oder aber so zu beeinflussen, dass die Halterung 7 mit einer langsameren, definierten Vorschubbewegung verfahren wird.

## Patentansprüche

1. Injektionseinrichtung (1) mit
- einem Kolben (3) zur Verlagerung eines Stopfens in einer Spritze oder Karpule,
- einer Halterung (7) für den Kolben (3),
- einem Antrieb zur axialen Verlagerung der Halterung (7) des Kolbens (3),
- einer dem Antrieb zugeordneten Steuereinrichtung, wobei
- die Halterung (7) gegenüber dem Kolben (3) oder einem Teilelement (11) derselben entgegen einer Vorspannkraft über eine begrenzte Strecke relativ verlagerbar ist, wobei
- eine die Relativposition des Kolbens (3) oder des zumindest einen Teilelements (11) desselben gegenüber der Halterung (7) erfassende Sensoreinrichtung (27) vorgesehen ist,
- die wenigstens ein mit der Steuereinrichtung zusammenwirkendes Sensorelement (29) umfasst,
**dadurch gekennzeichnet, dass**
- die Steuereinrichtung so ausgelegt ist, dass bei Aktivierung des Sensorelementes (29) die Vorschubgeschwindigkeit der Halterung (7) des Kolbens (3) zumindest reduziert wird, wobei
- der Kolben (3) einen Grundkörper (9) umfasst, wobei das Teilelement (11) gegenüber diesem verlagerbar ist, und wobei
- der Grundkörper (9) des Kolbens (3) als ein einen Innenraum umfassender Hohlzylinder ausgebildet ist, und dass in dem Innenraum das als Schaltstößel ausgebildete Teilelement (11) verschieblich angeordnet ist.

2. Injektionseinrichtung (1) nach Anspruch 1, **dadurch gekennzeichnet, dass** das Sensorelement (29) einen Mikroschalter umfasst.

3. Injektionseinrichtung (1) nach Anspruch 2, **dadurch gekennzeichnet, dass** der Mikroschalter von dem Kolben (3) oder von dem Teilelement (11) desselben betätigbar ist.

4. Injektionseinrichtung (1) nach Anspruch 3, **dadurch gekennzeichnet, dass** der Mikroschalter bei Erreichen einer definierten Relativposition des Kolbens (3) oder des Teilelements (11) davon bezüglich der Halterung (7) betätigbar ist.

5. Injektionseinrichtung (1) nach einem der vorhergehenden Ansprüche, **dadurch gekennzeichnet, dass** der Halter (7) ein Federelement (19, 23, 37) aufweist, welches den Kolben (3) oder das mindestens ein Teilelement (11) in eine erste Funktionsstellung drängt.

6. Injektionseinrichtung (1) nach Anspruch 5, **dadurch gekennzeichnet, dass** der Kolben (3) so an der Halterung (7) angebracht ist, dass er oder dessen Teilelement (11) bei Berührung eines Stopfens in einer Spritze oder Karpule gegen die Kraft des Federelementes aus der ersten Funktionsstellung verlagert wird.

7. Injektionseinrichtung (1) nach einem der vorhergehenden Ansprüche, **dadurch gekennzeichnet, dass** die Steuereinrichtung so ausgelegt ist, dass bei Aktivierung des Sensorelementes (29) der Antrieb der Injektionseinrichtung angehalten wird und damit die Halterung (7) des Kolbens (3).

8. Injektionseinrichtung (1) nach einem der vorhergehenden Ansprüche, **dadurch gekennzeichnet, dass** die Steuereinrichtung so ausgelegt ist, dass bei Aktivierung des Sensorelementes (29) der Vorschub der Halterung (7) des Kolbens (3) angehalten und dann mit reduzierter Geschwindigkeit weiter verlagert wird.

## Claims

1. An injection device (1), having
- a plunger (3) for displacing a stopper in a syringe or carpule,
- a holder (7) for the plunger (3),
- a drive for the axial displacement of the holder (7) of the plunger (3),
- a control device functionally assigned to the drive, wherein
- the holder (7) is able to move over a limited distance relative to the plunger (3) or a subcomponent (11) thereof, opposing a pretensioning force, in that
- a sensor device (27) is included which detects the relative position of the plunger (3), or at least a subcomponent (11) thereof, relative to the holder (7),
- which comprises at least one sensor element (29) which works together with the control device,
**characterized in that**
- the control device is designed in such a manner that, upon activation of the sensor element (29), the speed of advancement of the holder (7) of the plunger (3) is at least reduced, wherein
- the plunger (3) comprises a basic element (9), wherein the subcomponent (11) is displaceable thereto, and wherein
- the basic element (9) of the plunger (3) is constructed as a hollow cylinder enfolding an interior space, and **in that** the subcomponent (11) is constructed as a shift plunger displaceably arranged in the interior space.

2. The injection device (1) according to claim 1, **characterized in that** the sensor element (29) comprises a microswitch.

3. The injection device (1) according to claim 2, **characterized in that** the microswitch can be actuated by the plunger (3) or by a subcomponent (11) thereof.

4. The injection device (1) according to claim 3, **characterized in that** the microswitch can be actuated when a defined relative position of the plunger (3) or of a subcomponent (11) thereof relative to the holder (7) is reached.

5. The injection device (1) according to one of the preceding claims, **characterized in that** the holder (7) comprises a spring element (19,23,37), which biases the plunger (3) or the at least one subcomponent (11) into a first functional position.

6. The injection device (1) according to claim 5, **characterized in that** the plunger (3) is mounted on the holder (7) in such a manner that it or its subcomponent (11), upon contact of a stopper in a syringe or carpule, is moved against the force of the spring element out of the first functional position.

7. The injection device (1) according to one of the preceding claims, **characterized in that** the control device is designed in such a manner that the drive of the injection device, and therefore the holder (7) of the plunger (3), are stopped when the sensor element (29) is activated.

8. The injection device (1) according to one of the preceding claims, **characterized in that** the control device is designed in such a way that, when the sensor element (29) is activated, the advancement of the holder (7) of the plunger (3) is stopped, and then the advancement is continued at a reduced speed.

## Revendications

1. Dispositif d'injection (1) avec
- un piston (3) pour le déplacement d'un bouchon dans une seringue ou cartouche,
- un support (7) pour le piston (3),
- un entraînement pour le déplacement axial du support (7) du piston (3),
- un dispositif de commande affecté à l'entraînement,
- le support (7) étant déplaçable de manière relative par rapport au piston (3) ou à un élément partiel (11) de celui-ci contre une force de précontrainte sur un trajet limité,
- un dispositif détecteur (27) enregistrant la position relative du piston (3) ou au moins de l'élément partiel (11) de celui-ci, étant prévu en face du support (7),
- comprenant au moins un élément détecteur (29) interagissant avec le dispositif de commande,
**caractérisé en ce que**
- le dispositif de commande est conçu de façon à ce que la vitesse d'avance du support (7) du piston (3) soit au moins réduite lors de l'activation de l'élément détecteur (29),
- le piston (3) comprenant un corps de base (9), l'élément partiel (11) étant déplaçable en face de ce dernier et
- le corps de base (9) du piston (3) étant conçu comme un cylindre creux comprenant un espace intérieur, et à ce que l'élément partiel (11) conçu comme poussoir de commande soit disposé de façon déplaçable dans l'espace intérieur.

2. Dispositif d'injection (1) conformément à la revendication n°1, **caractérisé en ce que** l'élément détecteur (29) comprend un microrupteur.

3. Dispositif d'injection (1) conformément à la revendication n°2, **caractérisé en ce que** le microrupteur est actionnable par le piston (3) ou par l'élément partiel (11) de celui-ci.

4. Dispositif d'injection (1) conformément à la revendication n°3, **caractérisé en ce que** le microrupteur est actionnable lorsqu'une position relative définie du piston (3) ou de l'élément partiel (11) de celui-ci est atteinte par rapport au support (7).

5. Dispositif d'injection (1) conformément à l'une des revendications précédentes, **caractérisé en ce que** le support (7) présente un élément ressort (19, 23, 37) qui repousse le piston (3) ou au moins l'élément partiel (11) dans une première position de fonctionnement.

6. Dispositif d'injection (1) conformément à la revendication n°5, **caractérisé en ce que** le piston (3) est placé sur le support (7) de façon à ce qu'il ou son élément partiel (11) soit déplacé de la première position de fonctionnement dans une seringue ou cartouche contre la force de l'élément ressort lors du contact d'un bouchon.

7. Dispositif d'injection (1) conformément à l'une des revendications précédentes, **caractérisé en ce que** le dispositif de commande est conçu de façon à ce que l'entraînement du dispositif d'injection soit arrêté lors de l'activation de l'élément détecteur (29) et, par conséquent, le support (7) du piston (3).

8. Dispositif d'injection (1) conformément à l'une des revendications précédentes, **caractérisé en ce que** le dispositif de commande est conçu de façon à ce que l'avance du support (7) du piston (3) soit arrêtée lors de l'activation de l'élément détecteur (29), puis continue d'être déplacée à vitesse réduite.
